# EUROPEAN PATENT APPLICATION

(11) **EP 2 798 997 A1**
(43) Date of publication of application: **05.11.2014**
(21) Application number: 12861134.0
(22) Date of filing: 28.11.2012
(51) Int. Cl.: A61B 1/00, A61B 17/00

(54) **BODY CAVITY INTERNAL PRESSURE ADJUSTMENT DEVICE AND ENDOSCOPE SYSTEM**

(30) Priority: 28.12.2011 JP 2011288523
(71) Applicant: Olympus Medical Systems Corp., Tokyo 151-0072 (JP)
(72) Inventor: HIRAGA, Kunitoshi, Tokyo 151-0072 (JP); UESUGI, Takefumi, Tokyo 151-0072 (JP); KASUYA, Yuma, Tokyo 151-0072 (JP)
(74) Representative: Schicker, Silvia
(86) International application number: PCT/JP2012/080791
(87) International publication number: WO 2013/099507

(57) **Abstract**

A body cavity internal pressure regulating device 8 is connected to an endoscope 1 including a suction channel 17 used for suction under manual control and a forceps channel 19 used to insert an instrument into a body cavity, and the body cavity internal pressure regulating device regulates a body cavity internal pressure. A gas supply unit 24 performs a gas supply through the forceps channel 19. A pressure gauge 21 and a flowmeter 22 detects that suction is being performed under manual control through the suction channel 17. A control circuit 25 terminates the gas supply being performed by the gas supply unit according to a result of detection by the suction detection unit when the control circuit 25 detects that suction is being performed under manual control through the suction channel 17 while the gas supply unit 24 is performing a gas supply.

## Description

### TECHNICAL FIELD

The present invention relates to a body cavity internal pressure regulating device for regulating pressure inside a body cavity, which is connected to an endoscope apparatus having a suction channel used for suction under manual control and a forceps channel used to insert an instrument into a body cavity, and relates to an endoscope system having the body cavity internal pressure regulating device.

### BACKGROUND ART

When the inside of a body cavity is examined or treated by using an endoscope, it is necessary to supply gas externally to the inside of the body cavity such that the body cavity interior wall will expand appropriately due to the pressure applied by the gas and thereby an observation field will be secured. For this purpose, an endoscope system generally includes a gas supply unit that supplies gas into a body cavity, and a suction unit that sucks gas from the inside of the body cavity. An operator regulates the body cavity internal pressure by manipulating a control unit of the endoscope and manually controlling the gas supply unit and the suction unit. In addition to regulating the body cavity internal pressure, the suction may also be performed under manual control in order to remove dirt or the like, which is sucked up together with the gas from the inside of a body cavity.

Conventionally, endoscopes have mainly been used for examination, but in recent years, manipulation with endoscopes has become widespread. Accordingly, the operations performed by a doctor during the manipulation are becoming more complicated, and the burden is also increasing. Under these circumstances, it is desired that the operations of endoscope-related devices be simplified so as to reduce the burden on a doctor, and there has been a desire for the automation of the regulation of the body cavity internal pressure by a gas supply device for endoscopes.

To address this problem, a body cavity internal pressure regulating device that includes, in addition to the gas supply unit and suction unit under manual control, the second suction unit that performs automatic suction has been disclosed (see, for example, Patent Document 1). According to this disclosure, the body cavity internal pressure regulating device includes the second suction unit, a pressure sensor that measures the body cavity internal pressure and a control unit that controls the second suction unit, and the body cavity internal pressure regulating device performs automatic suction by controlling the second suction unit according to the body cavity internal pressure detected by the pressure sensor at the time of endoscope examination or treatment.

Furthermore, a technique in which the burden on an operator is further reduced in the regulation of the body cavity internal pressure by automatically performing not only the suction but also the gas supplying has also been disclosed (see, for example, Patent Documents 2, 3, and 4) . According to this disclosure, an air supply, a water supply, and suction with a high precision are achieved as the amount of supplied air, the amount of supplied water, and the amount of suction of the sucked up substance are detected, and as an air supply mechanism, a water supply mechanism, and a suction mechanism are controlled.

### PRIOR ART DOCUMENTS

### PATENT DOCUMENTS

[Patent Document 1] Japanese Laid-open Patent Publication No. 03-024844
[Patent Document 2] Japanese Laid-open Patent Publication No. 2007-020798
[Patent Document 3] Japanese Laid-open Patent Publication No. 2010-088572
[Patent Document 4] Japanese Laid-open Patent Publication No. 2006-181108

### SUMMARY OF INVENTIONTECHNICAL PROBLEMS

The ducts of an endoscope are composed of a gas-supply and water-supply channel, a suction channel, and a forceps channel, and the forceps channel intersects with the suction channel at some midpoint in the duct. Generally, gas supplying is performed through the gas-supply and water-supply channel when a gas supply is performed through an endoscope, and it is necessary for the body cavity internal pressure regulating device to supply gas at high pressure because the channel diameter is small. In such cases, the pressure resistance of the endoscope needs to be designed to be high because gas supplying is performed at high pressure. In resolving such problems, gas is fed through a forceps channel whose diameter is larger than that of the gas-supply and water-supply channel.

However, if the operator performs suction under manual control for the purpose of removing the dirt or the like inside the body cavity while the body cavity internal pressure regulating device is performing a gas supply under automatic control, the gas supplied by the body cavity internal pressure regulating device is sucked up in the above configuration because the forceps channel intersects with the suction channel. This may pose a difficulty in the removal of the dirt or the like inside the body cavity.

### SOLUTION TO PROBLEMS

A body cavity internal pressure regulating device in one aspect of the present invention is for regulating a body cavity internal pressure, and is connected to an endoscope device including a suction channel used for suction under manual control and a forceps channel used to insert an instrument into a body cavity, the body cavity internal pressure regulating device including: a gas supply unit for performing a gas supply through the forceps channel; a suction detection unit for measuring a body cavity internal pressure and a gas flow in the forceps channel; and a control unit for controlling gas supply by the gas supply unit according to a result of detection by the suction detection unit, and for terminating gas supply by the gas supply unit when the control unit detects, according to a body cavity internal pressure and a gas flow in the forceps channel calculated by the suction detection unit, that suction is being performed under manual control through the suction channel while the gas supply unit is performing a gas supply.

An endoscope system in one aspect of the present invention includes a suction device for performing suction by a suction operation under manual control, an endoscope device including a suction channel used for suction under manual control and a forceps channel used to insert an instrument into a body cavity, and a body cavity internal pressure regulating device connected to the endoscope device for regulating a body cavity internal pressure, and the body cavity internal pressure regulating device includes: a gas supply unit for performing a gas supply through the forceps channel; a suction detection unit for measuring a body cavity internal pressure and a gas flow in the forceps channel; and a control unit for controlling gas supply by the gas supply unit according to a result of detection by the suction detection unit, and for terminating gas supply by the gas supply unit when the control unit detects, according to a body cavity internal pressure and a gas flow in the forceps channel calculated by the suction detection unit, that suction is being performed under manual control through the suction channel while the gas supply unit is performing a gas supply.

### ADVANTAGEOUS EFFECT OF INVENTION

According to the present invention, it becomes possible to reduce the burden on an operator in relation to the regulation of the body cavity internal pressure, and it also becomes possible to avoid a malfunction caused when an operator performs suction under manual control while a body cavity internal pressure regulating device that automatically performs gas supplying and suction is operating.

### BRIEF DESCRIPTION OF DRAWINGS

FIG. 1 illustrates the usage environment of an endoscope system having a body cavity internal pressure regulating device according to an embodiment of the present invention.
FIG. 2 schematically illustrates the structure of ducts in an endoscope system.
FIG. 3 illustrates the structure of a forceps channel and a suction channel of an endoscope according to the first embodiment.
FIG. 4 is a timing chart in which the states of components of the body cavity internal pressure regulating device according to the first embodiment are illustrated in relation to the regulation of the body cavity internal pressure.
FIG. 5 is a flowchart illustrating a pressure regulation process performed by a control circuit.
FIG. 6 is a flowchart illustrating how the control circuit of a body cavity internal pressure regulating device according to the first embodiment performs control in each phase.
FIG. 7 is a block diagram illustrating a body cavity internal pressure regulating device according to the second embodiment.
FIG. 8 is a flowchart illustrating the control processes in each phase by a control circuit of a body cavity internal pressure regulating device according to the third embodiment.
FIG. 9 schematically illustrates the structure of ducts in an endoscope system according to the fourth embodiment.
FIG. 10 is a timing chart in which the states of components of the body cavity internal pressure regulating device according to the fourth embodiment are illustrated in relation to the regulation of the body cavity internal pressure.
FIG. 11 is a flowchart illustrating the control processes in each phase performed by a control circuit of a body cavity internal pressure regulating device according to the fourth embodiment.
FIG. 12 schematically illustrates another example of the structure of ducts in an endoscope system.

### DESCRIPTION OF EMBODIMENTS

Some embodiments of the present invention will be described in detail with reference to the drawings.

### <First Embodiment>

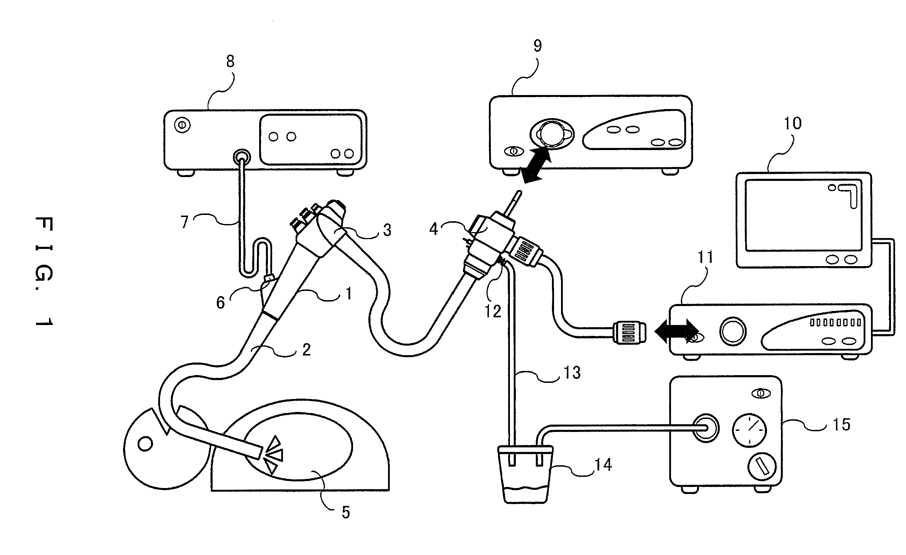

FIG. 1 illustrates the usage environment of an endoscope system having a body cavity internal pressure regulating device according to an embodiment of the present invention. As illustrated in FIG. 1, an endoscope system includes an endoscope 1, a monitor 10, a video processing device 11, a light source device 9, a body cavity internal pressure regulating device 8, and a suction device 15 in the present embodiment.

The endoscope 1 includes an insertion unit 2, a operation unit 3, and a connector unit 4. The insertion unit 2 is a portion that is inserted into a body cavity 5 of a patient, and a member of an optical system such as an image pickup device is integrated at the front end of the insertion unit 2. The operation unit 3 is provided with several kinds of electrical or mechanical control buttons for manipulating the endoscope 1. The operation unit 3 is also provided with a forceps insertion slot 6 on the periphery.

The body cavity internal pressure regulating device 8 is connected to a forceps channel of the endoscope 1 through a gas tube 7 connected to the forceps insertion slot 6, and regulates the body cavity internal pressure by supplying or sucking gas through the forceps channel.

The connector unit 4 is connected to the light source device 9 or the video processing device 11. The light source device 9 includes a lamp for illumination, and supplies illumination light to the endoscope 1. The video processing device 11 is provided with a means for communicating with the monitor 10 used to observe an endoscopic image, and performs image processing for an endoscopic image obtained by using the endoscope 1.

The connector unit 4 includes a suction inlet 12 at the lower end. The duct of the endoscope 1 is connected to the suction device 15 through a suction bottle 14 and a suction tube 13 that is connected to the endoscope 1 at the suction inlet 12.

In the endoscope system according to the present embodiment, the body cavity internal pressure regulating device 8 regulates the body cavity internal pressure by supplying or sucking gas through a forceps channel. In addition to the suction or the like performed by the body cavity internal pressure regulating device 8, an operator is required to manually perform the suction so as to remove dirt or the like when the inside of a body cavity is examined or treated by using the endoscope 1. The suction device 15 is used by an operator to manually perform suction, and the suction device 15 sucks up the gas inside the body cavity or the dirt or the like inside the body cavity through a suction channel. The dirt or the like inside the body cavity sucked up by the suction device 15 together with the gas is collected in the suction bottle 14.

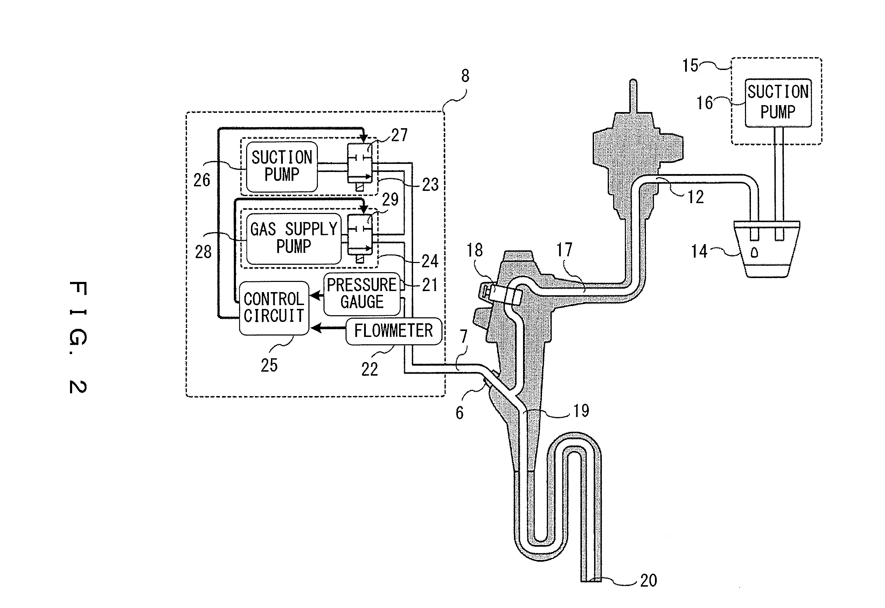

FIG. 2 schematically illustrates the structure of ducts in the endoscope system of FIG. 1. The same reference signs will be assigned to the components of FIG. 2 that are similar to the components of FIG. 1. The same applies to the drawings described below.

The suction device 15 is integrated with a suction pump 16. The suction pump 16 is connected to the suction inlet 12 via the suction bottle 14, and is connected to a suction channel 17 inside the endoscope 1. The suction channel 17 is connected to a forceps channel 19 through a manual valve 18 of the operation unit 3. The forceps channel 19 has apertures at the forceps insertion slot 6 on the periphery of the operation unit 3 and a front end 20 of the endoscope 1, and thus an instrument may be inserted through the forceps insertion slot 6 when the inside of a body cavity is examined or treated by using the endoscope 1. In the embodiment, the body cavity internal pressure regulating device 8 is connected to the forceps channel 19 through the forceps insertion slot 6.

The body cavity internal pressure regulating device 8 includes a suction unit 23, a gas supply unit 24, a pressure gauge 21, a flowmeter 22, and a control circuit 25, and regulates the body cavity internal pressure by supplying gas to the inside of a body cavity or by sucking gas from the inside of a body cavity through the forceps channel 19.

The suction unit 23 includes a suction pump 26 and a solenoid valve 27. The suction pump 26 is always operating and negative pressure is applied thereto. When the solenoid valve 27 is opened, the suction pump 26 sucks the gas inside the body cavity through the forceps channel 19.

The gas supply unit 24 includes a gas supply pump 28 and a solenoid valve 29. The gas supply pump 28 is always operating and positive pressure is applied thereto. When the solenoid valve 29 is opened, the gas supply pump 28 supplies gas to the inside of a body cavity through the forceps channel 19.

The pressure gauge 21 measures the body cavity internal pressure, and the flowmeter 22 measures the flow of gas in the ducts.

When the value measured by the pressure gauge 21 (hereinafter, referred to by measured pressure) is less than a specified value of pressure (hereinafter, referred to as set pressure) or greater than the set pressure, the control circuit 25 controls the solenoid valve 29 of the gas supply unit 24 or the solenoid valve 27 of the suction unit 23 so as to open or close.

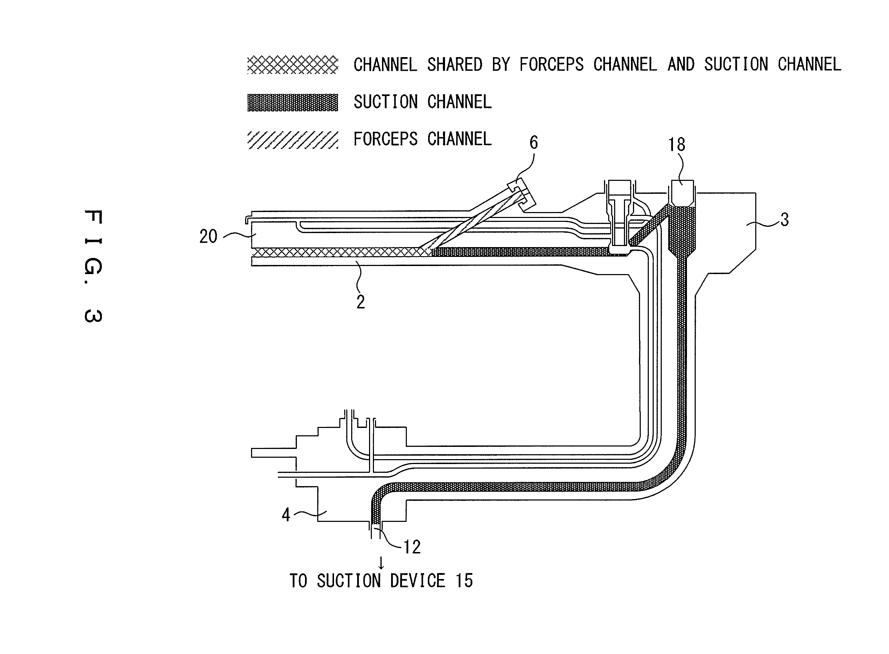

Moreover, the control circuit 25 determines that the suction device 15 of FIG. 1 is performing suction through the suction channel 17 according to the pressure and flow rate measured by the pressure gauge 21 and the flowmeter 22. When it is determined that the suction device 15 is performing suction while the gas supply unit 24 is supplying gas according to the results of the measurement by the pressure gauge 21 and the flowmeter 22, the control circuit 25 controls the gas supply unit 24 that supplies gas. This is because the forceps channel 19 that is connected to the body cavity internal pressure regulating device 8 through the forceps insertion slot 6 partly shares the duct with the suction channel 17 connected to the suction device 15. The relationship between the control of the gas supply/suction performed by the body cavity internal pressure regulating device 8 according to the present embodiment and the structure of the ducts of the endoscope 1 will be described with reference to FIG. 3.

FIG. 3 illustrates the structure of the forceps channel 19 and the suction channel 17 of the endoscope 1 according to the present embodiment.

As described above, the body cavity internal pressure regulating device 8 is connected to the forceps channel 19 of the endoscope 1 through the forceps insertion slot 6, and the body cavity internal pressure regulating device 8 supplies gas to or sucks gas out through the forceps channel 19 under automatic control. On the other hand, the suction device 15 is connected to the suction channel 17 of the endoscope 1 through the suction inlet 12 of the connector unit 4, and the suction device 15 sucks gas through the suction channel 17 under manual control.

The suction device 15 sucks the dirt inside the body cavity from the forceps channel 19 through the suction channel 17 by using the suction force caused by the suction pump 16 arranged inside. Normally, the suction channel 17 is separated from the forceps channel 19 by the manual valve 18 of the operation unit 3, and thus the suction is not performed by the suction device 15. When an operator presses the manual valve 18 of the operation unit 3, the valve opens and the suction channel 17 is connected to the forceps channel 19 in the way of a duct, and the suction is enabled.

As illustrated in FIG. 3, the forceps channel 19 and the suction channel 17 of the endoscope 1 share the duct at a portion of the insertion unit 2. For this reason, when the suction device 15 sucks gas out through the suction channel 17 due to the suction under manual control, the gas within the duct of the body cavity internal pressure regulating device 8 at which the forceps channel 19 of the endoscope 1 is connected to the duct through the forceps insertion slot 6 is also sucked out. Accordingly, the body cavity internal pressure regulating device 8 detects the flow of gas toward the outside of the device so as to detect the decreasing pressure inside the duct when the suction is performed under manual control.

As described above, when the body cavity internal pressure regulating device 8 detects the decreasing pressure inside the duct, the body cavity internal pressure regulating device 8 supplies gas by using the gas supply unit 24. However, when the body cavity internal pressure regulating device 8 supplies gas while the operator executes the suction under manual control by manipulating the manual valve 18 of the control unit 3, the suction force under manual control decreases and the removal of the dirt or the like may be affected.

Hence, the control circuit 25 of the body cavity internal pressure regulating device 8 according to the present embodiment not only compares the measured pressure with a set pressure and controls the gas supply and suction such that the body cavity internal pressure will be maintained constant, but also recognizes that the suction device 15 is performing the suction according to the values measured by the flowmeter 22 and the pressure gauge 21 and controls the gas supply and suction accordingly. Hereinafter, a method of controlling the gas supply and suction by the body cavity internal pressure regulating device 8 will be described in a specific manner.

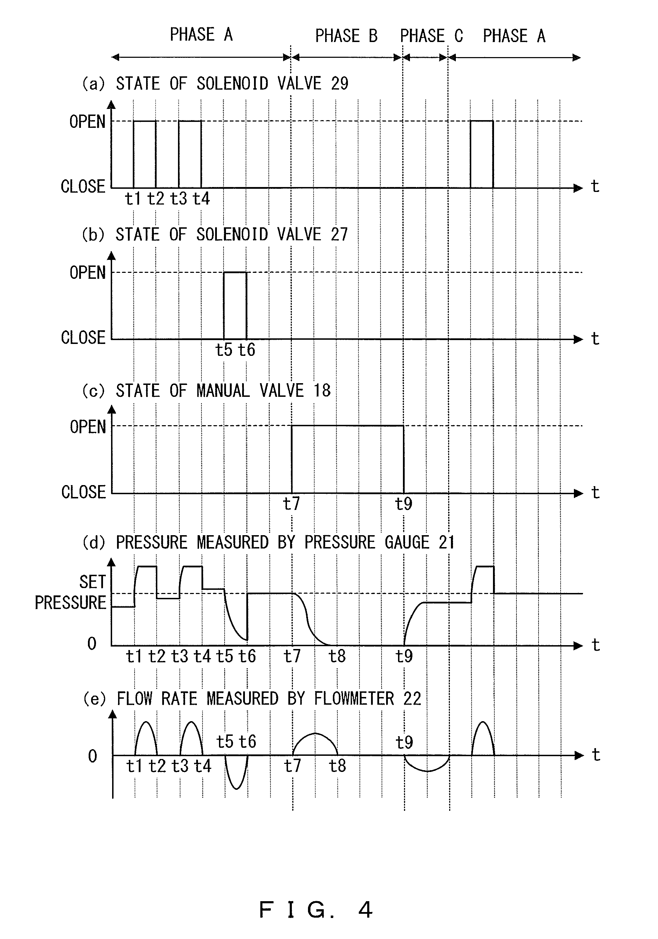

FIG. 4 is a timing chart in which the states of components of the body cavity internal pressure regulating device 8 according to the present embodiment are illustrated in relation to the regulation process of the body cavity internal pressure. In the timing chart of FIG. 4, the vertical axis indicates the states of each component, and the horizontal axis indicates the time.

Firstly, how the pressure gauge 21, the flowmeter 22, the solenoid valve 27 of the suction unit 23, and the solenoid valve 29 of the gas supply unit 24 of the body cavity internal pressure regulating device 8, and the manual valve 18 arranged at the control unit 3 of the endoscope 1 operate will be described with reference to FIG. 4, in relation to the regulation of the body cavity internal pressure.

In the following description, it is assumed that the period during which processes are performed so as to maintain the body cavity internal pressure at a specified set pressure is phase A, the period during which suction is being performed by an operator under manual control is phase B, and the period up to the time at which the process in phase A starts after the suction under manual control is complete is phase C.

In phase A, the solenoid valve 29 of the gas supply unit 24 or the solenoid valve 27 of the suction unit 23 are opened or closed depending on whether the value measured by the pressure gauge 21 is high or low with reference to a set pressure, and the gas supply or suction are performed. In the following description, the process in which gas supply or suction are performed so as to maintain the body cavity internal pressure in phase A at a set pressure will be referred to as a body cavity internal pressure regulation process.

As illustrated in FIG. 4(d) for example, the body cavity internal pressure measured by the pressure gauge 21 is lower than the set pressure in the initial state (time t=0). For this reason, the solenoid valve 29 of the gas supply unit 24 is left open for a specified period from time t1, as illustrated in FIG. 4(a). The flowmeter 22 detects the flow of gas caused by the gas supply, i.e., the flow of the gas from the body cavity internal pressure regulating device 8 to the endoscope 1, and the value measured by the pressure gauge 21 increases (see time t1 to t2 in FIG. 4(d)(e)).

Here, it is to be noted that the value measured by the pressure gauge 21 during the gas supplying or suction is not equivalent to the value of body cavity internal pressure. For example, gas supply pressure is applied to the pressure gauge 21 during t1 to t2 where the solenoid valve 29 is opened and gas supplying is being performed, and the value measured by the pressure gauge 21 temporarily increases. However, such a rise in the pressure is only temporary, and it becomes possible to accurately measure the body cavity internal pressure again when the solenoid valve 29 is closed.

The flowmeter 22 detects the flow of gas while the solenoid valve 29 or the solenoid valve 27 is open. Note that some pressure gauges are only capable of measuring the one-way flow, but the flowmeter 22 of the body cavity internal pressure regulating device 8 according to the present embodiment is required to detect at least the flow of gas when gas is being supplied. In the embodiment, a flowmeter 22 is used which is capable of detecting the flow of gas both when gas is being supplied and when suction is being performed as illustrated in FIG. 4(e), and it is assumed that the direction in which the flow of gas when gas is being supplied is the normal direction.

In the example of FIG. 4, the body cavity internal pressure measured by the pressure gauge 21 after the solenoid valve 29 is closed does not yet reach the set pressure, and thus gas is further supplied by the gas supply unit 24. The solenoid valve 29 is opened, and after a specified period t3 to t4 has passed, the solenoid valve 29 is closed. In FIG. 4, cases in which the body cavity internal pressure measured by the flowmeter 22 exceeds the set pressure afterward will be described as an example (see time t4 in FIG. 4(d)).

When the body cavity internal pressure exceeds the set pressure, the solenoid valve 27 of the suction unit 23 is opened, and suction is performed. As described above, suction force is applied while the solenoid valve 27 is opened and suction is being performed, and thus it is not possible to accurately measure the body cavity internal pressure in a similar manner to when gas is being supplied. However, in a similar manner to when gas is being supplied as above, it becomes possible to accurately measure the body cavity internal pressure also in the case of suction by measuring the pressure after the solenoid valve 27 is closed. In FIG. 4, cases in which the body cavity internal pressure has reached the set pressure by performing suction during a specified period t5 to t6 will be described as an example (see time t5 to t6 in FIG. 4(b), FIG. 4(d), and FIG. 4(e)).

In phase B, the suction device 15 performs the suction through the suction channel 17 during a period t7 to t9 where the manual valve 18 of the operation unit 3 is pressed and the manual valve 18 is in an opened state, as illustrated in FIG. 4(c).

In phase B, the flowmeter 22 detects the flow of gas as the gas accumulated in the duct of the body cavity internal pressure regulating device 8 is sucked up due to the suction performed by the suction device 15 even though the solenoid valve 29 of the gas supply unit 24 is closed (see time t7 to t8 in FIG. 4(e)). As the gas within the duct of the body cavity internal pressure regulating device 8 is also sucked up after the manual valve 18 is opened, the value measured by the pressure gauge 21 gradually decreases (see time t7 or later in FIG. 4(d)).

Because a pressure gauge 21 capable of measuring only positive pressure is used in the embodiment, the value measured by the pressure gauge 21 becomes "0" after the manual valve 18 is opened (see time t8 in FIG. 4(d)). It is to be noted that the actual value of the body cavity internal pressure is not zero, and in fact, negative pressure is caused inside the duct. This is because the suction force caused by the suction pump 16 within the suction device 15 acts inside the duct.

When the depression of the manual valve 18 of the operation unit 3 ceases at time t9 and the time enters the phase C, the value measured by the pressure gauge 21 begins to increase again because the suction by the suction device 15 has been terminated. This happens because the suction force that has been acting inside the duct is eliminated when the manual valve 18 is closed and then a portion of the gas inside the body cavity flows into the duct, and thereby the pressure becomes equal (equilibrated) between inside the body cavity and inside the duct. In phase C, the decreased pressure in phase B increases again, and thus the gas flows in the direction of entering the body cavity internal pressure regulating device 8 (i.e. , in the negative direction) (see time t9 or later in FIG. 4(e)). After the pressure is equilibrated, the body cavity internal pressure regulation process of phase A is performed in a similar manner to the above.

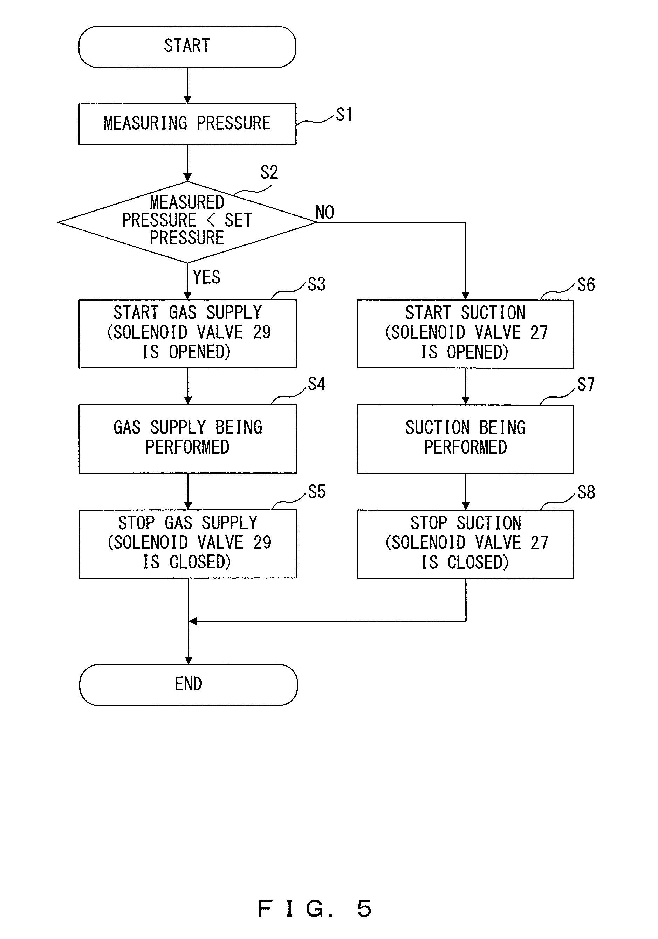

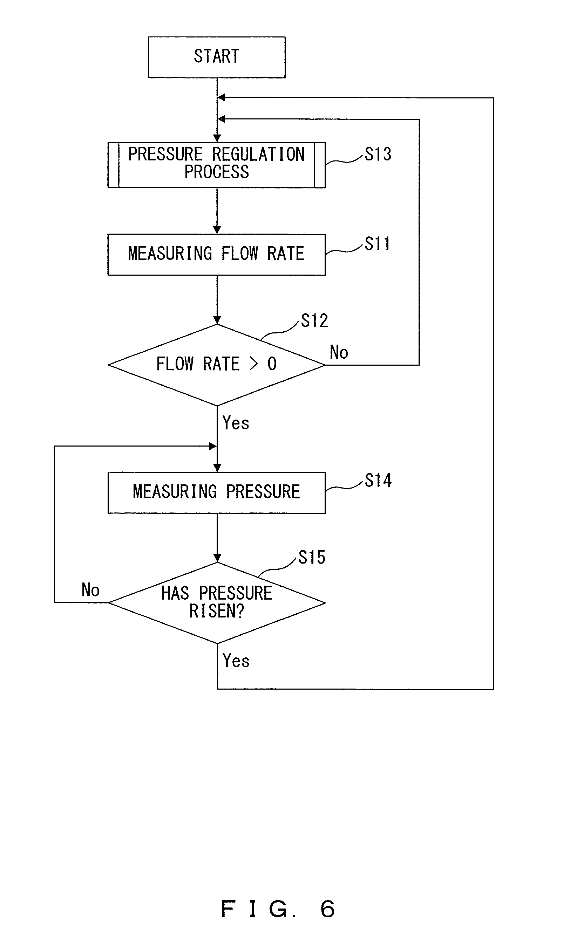

Next, a method for controlling components within the body cavity internal pressure regulating device 8 in phases A to C of FIG. 4 by the control circuit 25 will be described with reference to FIG. 5 and FIG. 6.

FIG. 5 is a flowchart illustrating a pressure regulation process performed by the control circuit 25. For example, the control circuit 25 of the body cavity internal pressure regulating device 8 constantly performs the process illustrated in FIG. 5.

Firstly, the control circuit 25 obtains a measured value from the pressure gauge 21 in step S1, and the control circuit 25 compares the measured pressure with a set pressure stored in a memory or the like (not illustrated in FIG. 2) in step S2.

When it is determined that the measured value is smaller than the set pressure in step S2, the control circuit 25 shifts the process to step S3. Then, in step S3, the control circuit 25 controls the solenoid valve 29 of the gas supply unit 24 such that it will open, and in step S4, the control circuit 25 controls the gas supply unit 24 to supply gas for a specified period. In step S5, the control circuit 25 controls the solenoid valve 29 of the gas supply unit 24 such that it will close, and terminates the process.

When it is determined that the measured value is equal to or greater than the set pressure in step S2, the control circuit 25 shifts the process to step S6. Then, in step S6, the control circuit 25 controls the solenoid valve 27 of the suction unit 23 such that it will open, and in step S7, the control circuit 25 controls the suction unit 23 to perform suction for a specified period. In step S8, the control circuit 25 controls the solenoid valve 27 of the suction unit 23 such that it will close, and terminates the process.

When an operator is not performing suction under manual control and the pressure is equilibrated between inside the body cavity and inside the duct of the body cavity internal pressure regulating device 8, the control circuit 25 performs a series of processes of FIG. 5. Next, how the control circuit 25 performs control in phases A to C of FIG. 4 will be described in detail with reference to FIG. 6.

FIG. 6 is a flowchart illustrating how the control circuit 25 of the body cavity internal pressure regulating device 8 according to the present embodiment performs control in each phase. When, for example, the body cavity internal pressure regulating device 8 starts operating and an instruction to start a pressure regulation process is received, the control circuit 25 starts the processes of FIG. 6.

Firstly, as described above with reference to the flowchart of FIG. 5, a pressure regulation process of step S13 is performed. Subsequently, in step S11, the control circuit 25 obtains a measured value from the flowmeter 22, and in step S12, the control circuit 25 determines whether or not the flow of gas in the gas supplying direction has been detected inside the body cavity internal pressure regulating device 8. When the flow of gas in the gas supplying direction is not detected, i.e., when a positive measured value is not detected, the process is shifted to step S13.

When a pressure regulation process has been performed in step S13, the control circuit 25 returns the process to step S11.

On the other hand, when the flow of gas in the gas supplying direction (positive measured value) is detected in the determination of step S12, the control circuit 25 shifts the process to step S14.

In step S14, the control circuit 25 obtains a measured value from the pressure gauge 21, and in step S15, the control circuit 25 determines whether or not the pressure has risen by referring to the obtained measured value. Whether or not the pressure has risen is determined, for example, by comparing the measured value that has been obtained from the pressure gauge 21 and stored in a storage unit such as a memory in advance with the measured value obtained in step S14.

When no pressure rise is recognized in step S15, the process returns to step S14, and a similar process is repeated until the pressure starts increasing. This is because it can be determined that the flow of gas is not caused by the gas supply from the gas supply unit 24 but is caused by the suction performed by the suction device 15 when a flow in a positive direction is detected as the flow rate of the gas in step S12 and the pressure is not increasing. When a rise in pressure is detected in step S15, the control circuit 25 returns the process to step S11.

Note that the value measured by the flowmeter 22 will be a positive value while the gas supply unit 24 is performing a gas supply in a pressure regulation process. However, in a pressure regulation process for maintaining the body cavity internal pressure at a set pressure, gas supplying is performed only for a specified period in step S4 of FIG. 5, and the process is terminated after the solenoid valve 29 of the gas supply unit 24 is closed in step S5. For this reason, when a positive measured value is detected as a flow rate of the gas in step S11, it can be determined that such a positive measured value is not obtained due to the gas supply performed by the body cavity internal pressure regulating device 8.

When it is recognized that the pressure changes and starts increasing in the determination of step S15, it is determined that the phase in FIG. 4 shifts from phase B to phase C and the suction under manual control is complete.

As described above, according to the body cavity internal pressure regulating device 8 according to the present embodiment, it is detected that the suction is being performed through the suction channel 17 under manual control by referring to the values measured by the flowmeter 22 and the pressure gauge 21. When it is detected that the suction is being performed under manual control, the gas supply being performed by the gas supply unit 24 is controlled. In particular, the gas supply being performed by the gas supply unit 24 is terminated. The pressure within the duct decreases due to the suction being performed under manual control, but the suction force of the suction under manual control is prevented from decreasing by performing control such that the gas supply will not be performed when the detected pressure is recognized as is. By maintaining the body cavity internal pressure at a set value according to the measured pressure measured by the pressure gauge 21, the burden on an operator in relation to the regulation of the body cavity internal pressure is reduced, and a malfunction such as a difficulty in the removal of the dirt or the like inside the body cavity caused when an operator performs suction under manual control is avoided.

Further, the body cavity internal pressure regulating device 8 according to the present embodiment is configured to be connected to the forceps channel 19 through the forceps insertion slot 6. In the configuration of conventional endoscopes, a valve (e.g., the manual valve 18 of FIG. 2) that is used to perform gas supplying or suction under manual control is arranged at the operation unit 3 of FIG. 1. As the body cavity internal pressure regulating device 8 is configured to be connected via the forceps insertion slot 6 that is arranged on a side upstream to the operation unit 3, even in conventional endoscopes in which gas supplying and suction is manually performed by pressing a valve of the operation unit 3, it becomes possible to perform the control of the gas supply and suction without making changes to the configuration.

### <Second Embodiment>

In the embodiment described above, the direction of the flow of gas in the ducts is detected by using the flowmeter 22. By contrast, a differential pressure gauge is used instead of a flowmeter in the present embodiment, which makes it different.

Hereinafter, the body cavity internal pressure regulating device 8 according to the present embodiment will be described, mainly in regard to the points different from the above embodiment.

The usage environment of an endoscope system including the body cavity internal pressure regulating device 8 according to the present embodiment is similar to that of the above embodiment as illustrated in FIG. 1.

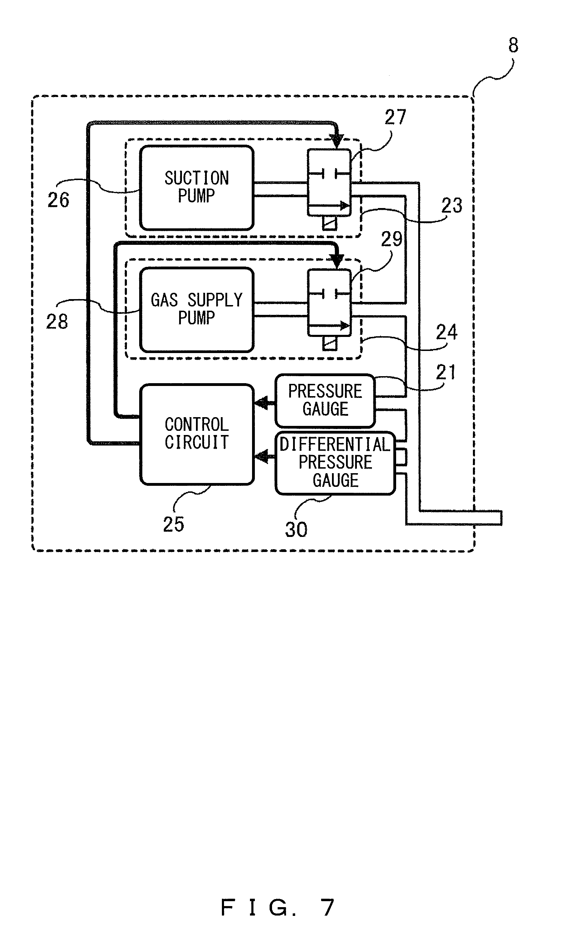

FIG. 7 is a block diagram illustrating the body cavity internal pressure regulating device 8 according to the present embodiment. In the drawing, like reference signs denote like components as in the embodiment described above. As the structure of the ducts of the endoscope 1 or the like is similar to that of the embodiment illustrated in FIG. 2 or FIG. 3, such a structure or the like is omitted in FIG. 7.

As described above, the body cavity internal pressure regulating device 8 according to the present embodiment is provided with a differential pressure gauge 30 instead of the flowmeter 22. The differential pressure gauge 30 includes two ports, and measures the pressure of the ports to detect the difference in pressure between two fixed points on the duct within the body cavity internal pressure regulating device 8. The control circuit 25 determines the flow of gas and the direction according to the detected difference in pressure.

The operations of components or the like in phases A to C of FIG. 4 when the body cavity internal pressure regulating device 8 of FIG. 7 is used are as described in the description of the above embodiment with reference to FIGs. 4-6.

According to the body cavity internal pressure regulating device 8 of the present embodiment, it becomes possible to detect suction under manual control and to control the gas supply or suction performed by the gas supply unit 24 and the suction unit 23, in a similar manner to the embodiment described above. Accordingly, advantageous effects similar to the above body cavity internal pressure regulating device 8 according to the first embodiment may be achieved.

### <Third Embodiment>

In the first and second embodiment described above, the detection of the completion of suction under manual control is not followed by any particular process. By contrast, gas supplying is performed in the present embodiment when the completion of suction under manual control is detected, which makes the present embodiment different.

The following description of the body cavity internal pressure regulating device 8 according to the present embodiment will focus on the differences from the above embodiments.

The usage environment of an endoscope system having the body cavity internal pressure regulating device 8 according to the present embodiment is similar to that of the first and second embodiments described above, and is as described above with reference to FIG. 1. The body cavity internal pressure regulating device 8 may be configured in a similar manner to any one of the first and second embodiments, and the operations of the components of the body cavity internal pressure regulating device 8 are as described above with reference to FIGs. 4-6.

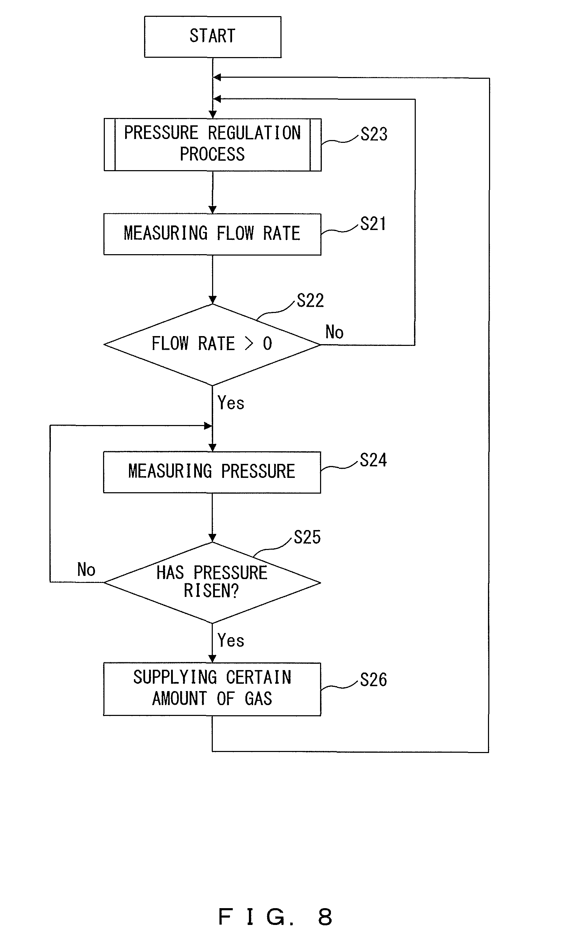

FIG. 8 is a flowchart illustrating the control processes in each phase by the control circuit 25 of the body cavity internal pressure regulating device 8 according to the present embodiment. When, for example, the body cavity internal pressure regulating device 8 starts operating and an instruction to start a pressure regulation process is received, the control circuit 25 starts the processes of FIG. 8.

The processes in steps S21 to S25 are similar to the processes in steps S11 to S15 of FIG. 6, respectively. A pressure regulation process in step S23 is as previously described with reference to FIG. 5.

When a rise in pressure is detected in step S25, the control circuit 25 shifts the process to step S26 and controls the gas supply unit 24 to perform a certain amount of gas supplying, and then returns the process to step S21.

Generally, the inside of the duct of the endoscope 1 is placed under a negative pressure due to the suction force caused by suction under manual control. After the suction under manual control is terminated, sometimes, the dirt accumulated in the forceps channel 19 is led by the negative pressure inside the gas tube 7 and flows into the body cavity internal pressure regulating device 8. To address this problem, in the present embodiment, when the completion of suction under manual control is detected in step S25, a certain amount of gas supplying is performed so as to prevent the inflow of the dirt.

In step S25, it is preferable that the gas equivalent to the capacity of the gas tube 7 be supplied. In the embodiment, for example, gas supplying on the order of 10cc is performed.

As described above, according to the body cavity internal pressure regulating device 8 according to the present embodiment, a specified amount of gas supplying is performed after the suction under manual control is terminated. Accordingly, in addition to advantageous effects similar to those achieved by the first and second embodiments above, it becomes further possible to avoid the situation in which the dirt accumulated in the forceps channel 19 or the like flows into the body cavity internal pressure regulating device 8 and contaminates the device.

### <Fourth Embodiment>

In the above first to third embodiments, one forceps channel is provided for the endoscope 1, and the forceps channel 19 to which the body cavity internal pressure regulating device 8 is connected shares the duct with the suction channel 17. By contrast, the endoscope 1 is provided with two forceps channels in the present embodiment, and the forceps channel to which the body cavity internal pressure regulating device 8 is connected through the forceps insertion slot does not share the duct with the suction channel, which makes the present embodiment different.

The following description will focus on the differences from the first to third embodiments.

The usage environment of an endoscope system having the body cavity internal pressure regulating device 8 according to the present embodiment is similar to that of the embodiments described above, and is as described above with reference to FIG. 1.

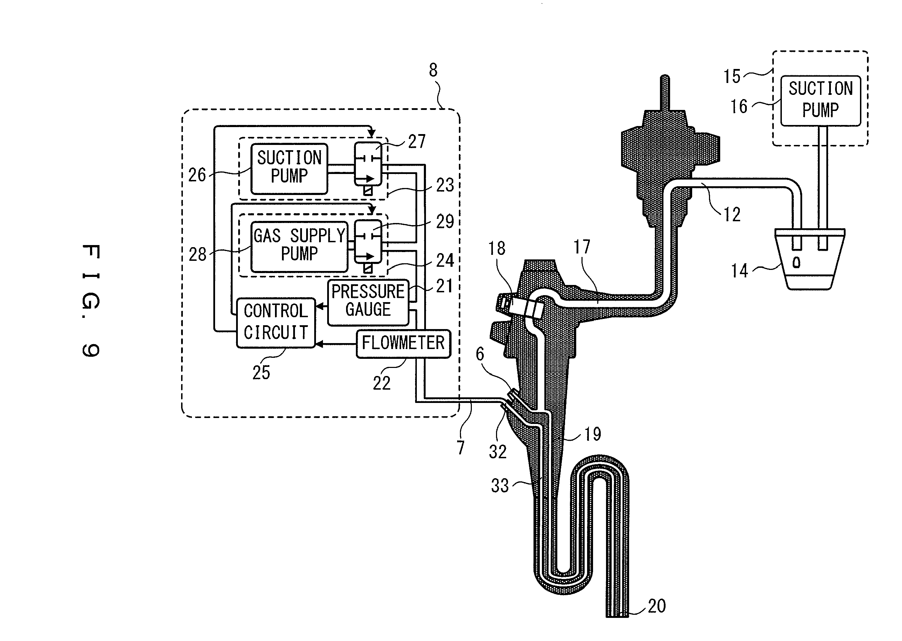

FIG. 9 schematically illustrates the structure of ducts in the endoscope system according to the present embodiment. In FIG. 9, like reference signs denote like components as in the embodiments described above.

Note that cases in which the body cavity internal pressure regulating device 8 is configured in a similar manner to the body cavity internal pressure regulating device 8 according to the first embodiment are illustrated in FIG. 9 as examples, but the present embodiment is not limited to such a configuration. For example, the differential pressure gauge 30 may be provided instead of the flowmeter 22 as in the body cavity internal pressure regulating device according to the second embodiment.

As described above, the endoscope 1 used in the present embodiment is a two-channel endoscope device for which two forceps channels are provided. Among the two forceps channels, the forceps channel 19 is directly connected to the suction channel 17, but a forceps channel 33 on the other side is not directly connected to the suction channel 17. The body cavity internal pressure regulating device 8 according to the present embodiment is connected to the forceps channel 33 through a forceps insertion slot 32 arranged on the endoscope 1.

Even in the cases where the body cavity internal pressure regulating device 8 is connected to the endoscope 1 of two-channel type through the forceps insertion slot 32, the gas in the duct of the body cavity internal pressure regulation device 8 is sucked. In other words, when suction is performed under manual control through the suction channel 17, the gas accumulated in the duct of the forceps channel 33 and the body cavity internal pressure regulating device 8 is also sucked through the body cavity. For this reason, when the suction is performed under manual control, the pressure measured by the pressure gauge 21 decreases. To address this problem, in a similar manner to the embodiments described above, the gas supplying and suction of the body cavity internal pressure regulating device 8 according to the present embodiment is also controlled according to the pressure gauge 21 and the value measured by the flowmeter 22 such that the gas supply unit 24 will not perform gas supplying while the suction is being performed under manual control.

How components of the body cavity internal pressure regulating device 8 are controlled and gas supplying and suction are performed when the body cavity internal pressure regulating device 8 according to the present embodiment is connected to the endoscope 1 with the structure of ducts as illustrated in FIG. 9 will be described in detail.

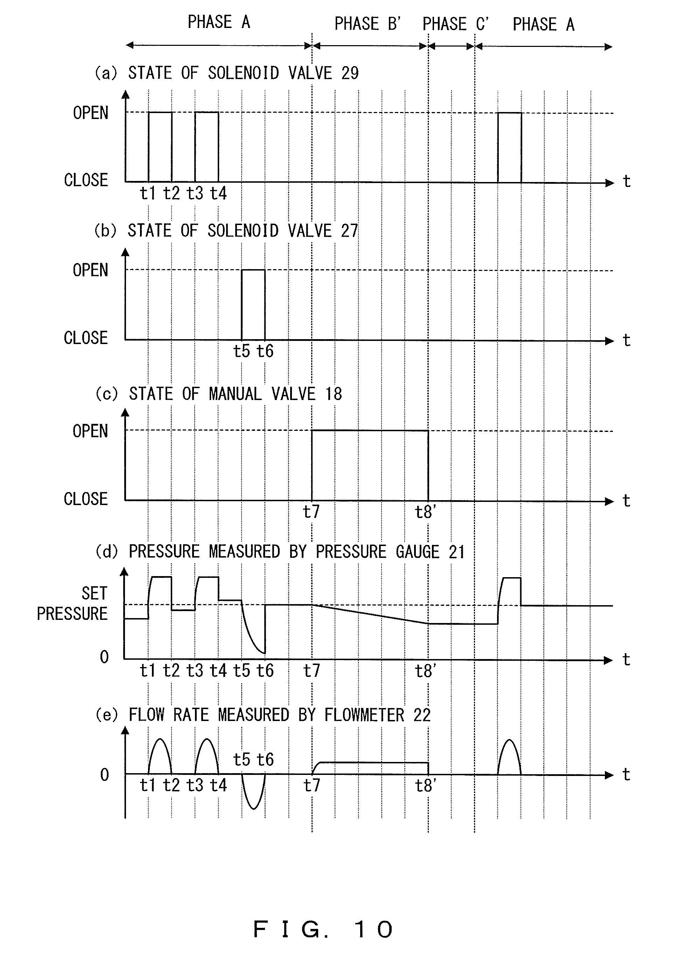

FIG. 10 is a timing chart in which the states of components of the body cavity internal pressure regulating device 8 according to the present embodiment are illustrated in relation to the regulation of the body cavity internal pressure. In a similar manner to FIG. 4, the vertical axis indicates the states of components, and the horizontal axis indicates the time. In FIG. 10, like reference signs denote like components as in the timing chart of FIG. 4.

Firstly, how each of the pressure gauge 21, the flowmeter 22, the solenoid valve 27 of the suction unit 23, and the solenoid valve 29 of the gas supply unit 24 of the body cavity internal pressure regulating device 8, and the manual valve 18 arranged at the operation unit 3 of the endoscope 1 operates will be described with reference to FIG. 10, in relation to the regulation of the body cavity internal pressure.

In a similar manner to the description of FIG. 4, it is assumed that the period in which a body cavity internal pressure regulation process is being performed is phase A. It is assumed that the period during which suction is being performed by an operator under manual control and the period up to the time at which the body cavity internal pressure regulation process in phase A starts after the suction under manual control is complete are referred to as phase B' and phase C', respectively, so as to be differentiated from the phases in the embodiment described above.

The operations of the body cavity internal pressure regulating device 8 in phase A (time t1 to t7) are as previously described with reference to FIG. 4.

In phase B', the suction device 15 performs suction through the suction channel 17 during the period t7 to t8' in which the manual valve 18 of the operation unit 3 is pressed and the manual valve 18 is in an opened state, in a similar manner to phase B (time t7 to t9) of FIG. 4.

Because the forceps channel 33 that is connected to the body cavity internal pressure regulating device 8 according to the present embodiment through the forceps insertion slot 32 is not connected to the suction channel 17 used to perform suction under manual control in the way of a duct, a suction force does not act on the forceps channel 33. Because a suction force does not act on the forceps channel 33 when the two-channel endoscope device is used, it is possible for the body cavity internal pressure regulating device 8 to accurately measure the body cavity internal pressure through the forceps channel 33 and the forceps channel 19 even when suction is being performed under manual control.

In particular, the value measured by the pressure gauge 21 does not become zero during the period in which the manual valve 18 of the operation unit 3 is being pressed by an operator (see time t7 to t8' in FIG. 10(c)), but the value measured by the pressure gauge 21 gradually decreases as the gas is sucked up by the suction device 15 (see time t7 to t8' in FIG. 10(d)).

When the gas inside the body cavity is sucked up through the suction channel 17, the gas accumulated in the duct of the body cavity internal pressure regulating device 8 connected to the forceps channel 33 is also sucked up because the forceps channel 33 also leads to the body cavity. For this reason, the flowmeter 22 detects a positive measured value during the period t7 to t8' in which the manual valve 18 is being pressed by an operator (see time t7 to t8' in FIG. 10(e)).

When the manual valve 18 is released at time t8' and the time enters phase C', the suction device 15 stops performing the suction and the body cavity internal pressure stops decreasing, and the value measured by the pressure gauge 21 becomes stable.

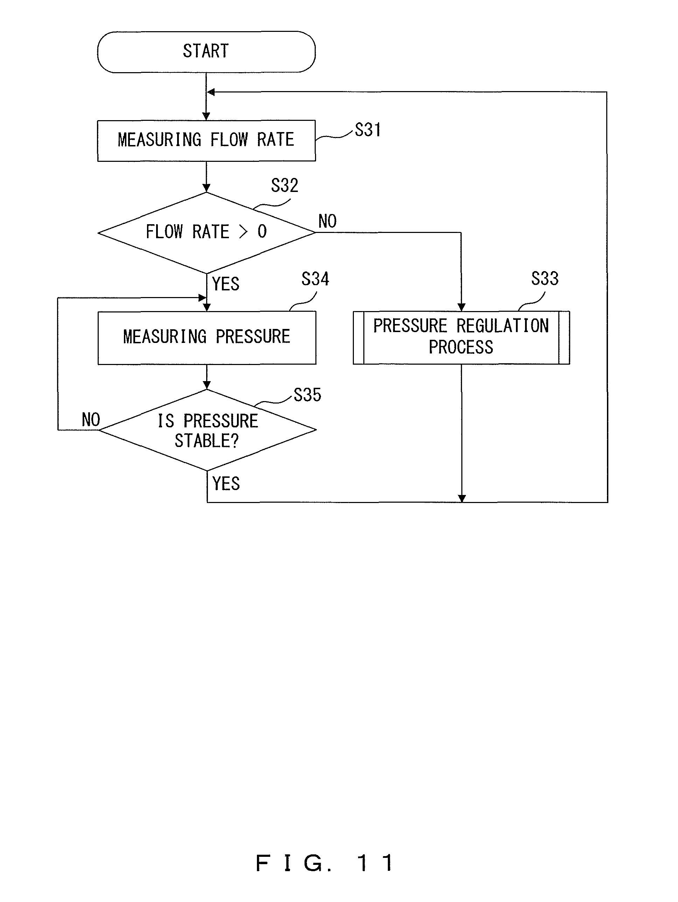

Next, a method for controlling components within the body cavity internal pressure regulating device 8 in phase A and phases B' to C' of FIG. 10 by using the control circuit 25 will be described with reference to FIG. 11.

FIG. 11 is a flowchart illustrating the control processes in each phase performed by the control circuit 25 of the body cavity internal pressure regulating device 8 according to the present embodiment. When, for example, the body cavity internal pressure regulating device 8 starts operating and an instruction to start a pressure regulation process is received, the control circuit 25 starts the processes of FIG. 11.

The processes in steps S31 to S33 are similar to the processes in steps S11 to S13 of FIG. 6, respectively. A pressure regulation process in step S33 is as previously described with reference to FIG. 5.

When the flow of gas in the gas supplying direction (i.e., a positive measured value) is detected by the flowmeter 22 in the determination of step S32, the control circuit 25 shifts the process to step S34. The determination in step S22 is based on the fact that a positive flow rate is measured by the flowmeter 22 when the manual valve 18 of the operation unit 3 is pressed and the suction is being performed through the suction channel 17, which is because the gas within the duct of the body cavity internal pressure regulating device 8 is gradually sucked up through the forceps channels 33 and 19.

The control circuit 25 obtains a measured value from the pressure gauge 21 in step S34, and determines whether or not the pressure is stable in step S35. Whether or not the pressure is stable may be determined, for example, by comparing the measured value obtained from the pressure gauge 21 in advance and stored in a storage unit such as a memory with the measured value obtained in step S34. When it is considered that the pressure stops decreasing as a result of the comparison, it is determined that the pressure has become stable.

When it is determined in step S35 that the pressure is not stable, the process returns to step S34, and repeats the processes in a similar manner until the pressure becomes stable. This is because it may be determined that the flow of gas is not caused by the gas supply performed by the gas supply unit 24 but is due to the suction being performed by the suction device 15 through the suction channel 17, if a positive flow rate is detected for the gas in step S32 and the pressure is not stable. When it is detected that the pressure is stable in step S35, the control circuit 25 returns the process to step S31.

An endoscope with two forceps channels is described by way of example in the above embodiment, but the configuration is not limited thereto. It is possible to perform control in a similar manner to the above with three or more forceps channels. In other words, when an operator performs suction under manual control, the body cavity internal pressure regulating device 8 detects decreasing pressure and the flow of gas in the normal direction. When decreasing pressure and the flow of gas in the normal direction is detected, it is determined that suction has started under manual control and the gas supply being performed by the gas supply unit 24 is terminated. When it is determined later that the suction under manual control is terminated as the pressure has become stable, a pressure regulation process is started again so as to maintain the body cavity internal pressure at a set value.

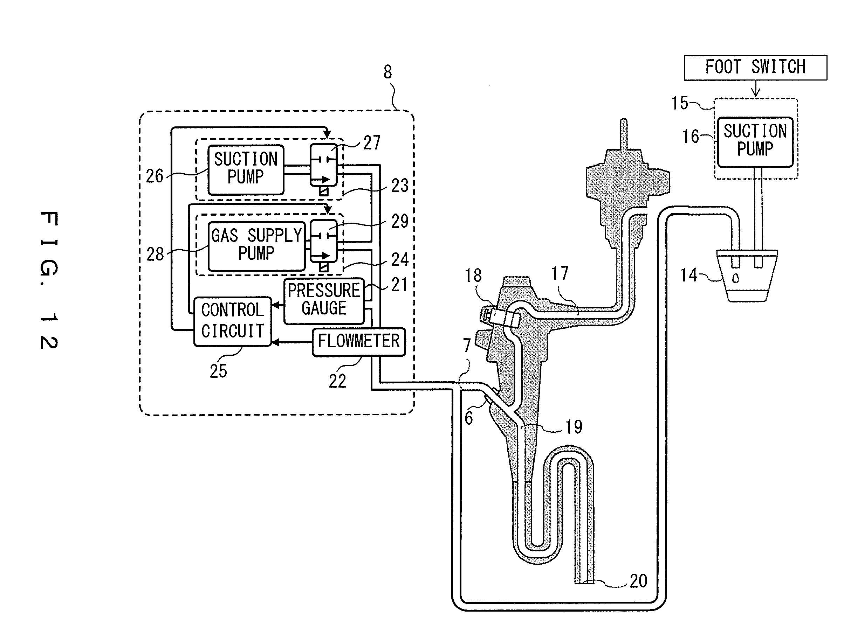

The suction device 15 is connected to the suction channel 17 of an endoscope in the embodiment described above, but the configuration is not limited thereto. For example, the suction device 15 may be connected to a forceps channel 6 of the endoscope through the gas tube 7 as illustrated in FIG. 12. In this case, suction is not performed via a suction button 18 of the endoscope, but is performed by depressing a foot switch connected to the suction device.

As described above, according to the body cavity internal pressure regulating device 8 of the present embodiment, it is possible to detect that suction is being performed under manual control through the suction channel 17 by referring to a result of detection by the pressure gauge 21 or the flowmeter 22 in a similar manner to the embodiments described above even when the body cavity internal pressure regulating device 8 is connected to the endoscope 1 with two or more forceps channels. Advantageous effects similar to those achieved by the above embodiments may be achieved by controlling the gas supply performed by the gas supply unit 24 according to a result of detection.

In addition to the above, various applications and modifications may be made without departing from the spirit or scope of the present invention. For example, some elements may be deleted from the overall configuration described in the embodiments above, or different elements of the embodiments may be combined as necessary.

## Claims

1. A body cavity internal pressure regulating device for regulating a body cavity internal pressure, the body cavity internal pressure regulating device being connected to an endoscope device including a suction channel used for suction under manual control and a forceps channel used to insert an instrument into a body cavity, the body cavity internal pressure regulating device comprising:
a gas supply unit for performing a gas supply through the forceps channel;
a suction detection unit for measuring a body cavity internal pressure and a gas flow in the forceps channel; and
a control unit for controlling gas supplying by the gas supply unit according to a result of detection by the suction detection unit, and for terminating gas supplying by the gas supply unit when the control unit detects, according to a body cavity internal pressure and a gas flow in the forceps channel calculated by the suction detection unit, that suction is being performed under manual control through the suction channel while the gas supply unit is performing a gas supply.

2. The body cavity internal pressure regulating device according to claim 1, wherein
the suction detection unit is arranged on a duct connecting the forceps channel and the gas supply unit.

3. The body cavity internal pressure regulating device according to claim 2, wherein
the suction detection unit includes a pressure gauge for measuring a body cavity internal pressure, and a flowmeter for measuring a gas flow in the duct, and
the control unit controls a gas supply by the gas supply unit when the flowmeter detects gas flow exiting the body cavity internal pressure regulating device and when the pressure gauge detects a reduction in body cavity internal pressure.

4. The body cavity internal pressure regulating device according to claim 2, wherein
the suction detection unit includes a pressure gauge for measuring a body cavity internal pressure and a differential pressure gauge for measuring a differential pressure in the duct, and
the control unit controls a gas supply by the gas supply unit when the differential pressure gauge detects a gas flow exiting the body cavity internal pressure regulating device and when the pressure gauge detects a reduction in body cavity internal pressure.

5. The body cavity internal pressure regulating device according to claim 3 or 4, wherein
the control unit controls the gas supply unit to stop performing a gas supply when the suction detection unit detects that suction is being performed through the suction channel.

6. The body cavity internal pressure regulating device according to claim 5, wherein
the control unit controls the gas supply unit to perform a specified amount of gas supplying when the control unit recognizes that suction by the suction detection unit through the suction channel is terminated after a gas supply by the gas supply unit is terminated.

7. The body cavity internal pressure regulating device according to claim 6, wherein
the gas supply unit includes a solenoid valve, and
the control unit controls the gas supply unit to terminate the gas supply or to perform a specified amount of gas supplying by controlling an opening and closing of the solenoid valve.

8. An endoscope system including a suction device for performing suction by a suction operation under manual control, an endoscope device including a suction channel used for suction under manual control and a forceps channel used to insert an instrument into a body cavity, and a body cavity internal pressure regulating device being connected to the endoscope device for regulating a body cavity internal pressure, the body cavity internal pressure regulating device comprising:
a gas supply unit for performing a gas supply through the forceps channel;
a suction detection unit for measuring a body cavity internal pressure and a gas flow in the forceps channel; and
a control unit for controlling a gas supply by the gas supply unit according to a result of detection by the suction detection unit, and for terminating a gas supplying by the gas supply unit when the control unit detects, according to a body cavity internal pressure and a gas flow in the forceps channel calculated by the suction detection unit, that suction is being performed under manual control through the suction channel while the gas supply unit is performing a gas supply.
